# FASCICULE DE BREVET EUROPEEN

(11) **EP 2 382 044 B1**
(45) Date de publication et mention de la délivrance du brevet: **01.08.2018**
(21) Numéro de dépôt: 09736848.4
(22) Date de dépôt: 12.10.2009
(51) Int. Cl.: B01J 31/22, C08G 18/22, C08G 18/48, C08G 18/76

(54) **NOUVEAUX CATALYSEURS POUR LA REACTION ENTRE UN ISOCYANATE ET UN ALCOOL**
NEUE KATALYSATOREN FÜR DIE UMSETZUNG VON EINEM ISOCYANAT UND EINEM ALKOHOL
NOVEL CATALYSTS FOR THE REACTION BETWEEN AN ISOCYANATE AND AN ALCOHOL

(30) Priorité: 13.10.2008 FR 0805637
(43) Date de publication de la demande: 02.11.2011
(73) Titulaire: ELKEM SILICONES France SAS, 69003 Lyon (FR)
(72) Inventeur: MALIVERNEY, Christian, F-69690 Saint Julien sur Bibost (FR); SAINT-JALMES, Laurent, F-69390 Vourles (FR)
(74) Mandataire: Mekki, Boualem
(86) Numéro de dépôt international: PCT/EP2009/007310
(87) Numéro de publication internationale: WO 2010/043354

(56) Documents cités:
- EP-A- 1 460 094
- EP-A- 1 634 903
- EP-A- 1 770 187
- EP-A- 1 878 493
- US-A1- 2008 207 938
- WERNER J. BLANK, Z.A. HE, E.T. HESSELL: "Catalysis of the isocyanate-hydroxyl reaction by non-tin catalysts" PROGRESS IN ORGANIC COATINGS, vol. 35, 1999, pages 19-29, XP002531500
- BABCOCK J R ET AL: "DEVELOPMENT OF IMPLEMENTATION OF NEW VOLATILE CD AND ZN PRECURSORS FOR THE GROWTH OF TRANSPARENT CONDUCTING OXIDE THIN FILMS VIA MOCVD" MATERIALS RESEARCH SOCIETY SYMPOSIUM PROCEEDINGS; [MATERIALS RESEARCH SOCIETY SYMPOSIUM PROCEEDINGS], MATERIALS RESEARCH SOCIETY, USA, vol. 623, 24 avril 2000 (2000-04-24), pages 317-328, XP009016966 ISBN: 978-1-55899-828-5

## Description

L'invention concerne de nouveaux catalyseurs pour la réaction entre un isocyanate et un alcool, étape clé pour la préparation de polymères polyuréthanes. Elle concerne plus spécifiquement l'utilisation de nouveaux catalyseurs qui ne soient pas à base d'étain.

Les polyuréthanes ont été initialement utilisés dans la fabrication de mousses et composés plastiques. Depuis, ces polymères se sont développés dans des domaines d'application très divers tels les élastomères, les thermoplastiques, les résines thermodurcissables, les systèmes expansés, les fibres textiles et les systèmes d'enduction (bains de couchage pour le papier, revêtements du bois, peintures automobiles, adhésifs,...).

Les polyuréthanes sont des polymères qui contiennent au moins un groupement uréthane (aussi appelé carbamate). Ce groupement est issu de la réaction entre un groupement alcool et un groupement isocyanate.

D'une manière générale, la synthèse d'un polyuréthane par réaction, non catalysée, d'un isocyanate avec un alcool primaire ou secondaire est conduite entre 50 et 100°C. Ainsi d'optimiser cette réaction, de nombreux catalyseurs ont déjà été proposés, par exemple les acides et les bases de Lewis, ainsi que de nombreux sels ou complexes métalliques. Des exemples de ces catalyseurs sont décrits dans les articles suivants:
- Gambiroza-Jukic, etal Kinetic analysis of bulk polymerization of diisocyanate and polyol; J. Appl. Polym. Sci., 1993, vol 47, p 513-519,
- Wong etal., Catalysis in competing isocyanate reactions, Competing phenyl isocyanate reaction catalyzed with N,N',N"-pentamethyldipropylenetriamine; J. Polym. Sci.; Part A, Polym. Chem. Ed., 1986, vol 24, p 2877-2890,
- Okada, H., etal The kinetics of the polyurethane-forming reaction between organic diisocyanates and glycols; Makromol. Chem., 1963, vol 66, p 91-101 et
- Blank, W J, etal Catalysis of the isocyanate-hydroxy reaction by non-tin catalysts.

EP1634903 nous enseigne l'utilisation d'un complexe de zinc de préférence l'octoate de zinc ou l'acetylacetonate de zinc comme catalyseur.

EP1878493 décrit l'utilisation d'un carbanion potentiellement de zinc comme catalyseur.

Les catalyseurs métalliques les plus utilisés sont les carboxylates d'alkylétain dont le plus connu est le dilaurate de dibutylétain. Cependant, les catalyseurs à base d'alkylétain, bien que très efficaces présentent l'inconvénient d'être toxiques (CMR-2: toxiques pour la reproduction).

Aussi cherche-t-on pour de nombreuses applications à les remplacer par des composés ne présentant pas ces inconvénients. En outre, l'industrie est toujours à l'affût de composés qui soient au moins aussi actifs que le dilaurate de dibutylétain, mais qui ne soient pas à base d'étain.

L'objectif essentiel de la présente invention est donc de fournir un catalyseur pour la réaction entre un isocyanate et un alcool qui soit au moins aussi actif que le dilaurate de dibutylétain, mais qui ne soit pas à base d'étain.

Un autre objectif essentiel de la présente invention est de fournir un catalyseur qui soit utilisable pour la synthèse des polyuréthanes.

Il a maintenant été trouvé, et c'est ce qui constitue l'objet de la présente invention un nouveau procédé pour préparer un composé **A** ayant au moins une fonction uréthane comprenant une étape 1) consistant à faire réagir un composé **B** ayant au moins une fonction isocyanate avec un composé **D** ayant au moins une fonction hydroxyle en présence d'une quantité catalytiquement efficace d'au moins un catalyseur **C** caractérisé en ce que ledit catalyseur **C** est un complexe ou sel métallique de formule **(1)** suivante:

[Zn(L¹)l₁(Y)ₓ] **(1)**

dans laquelle:
- l₁ = 2, et x= 1 ou 2,
- le symbole Y représente une amine ou une diamine, et
- le symbole L¹ est un anion β-dicétonato dérivé d'une β-dicétone de formule R¹COCHR²COR³ **(2)** et ladite β-dicétone est choisie parmi le groupe constitué par les β-dicétones: l'hexanedione-2,4; heptanedione-2,4; heptanedione-3,5; l'éthyl-3 pentanedione-2,4; méthyl-5 hexanedione-2,4; octanedione-2,4; octanedione-3,5; diméthyl-5,5 hexanedione-2,4; méthyl-6 heptanedione-2,4; diméthyl-2, 2 nonanedione-3,5; diméthyl-2,6 heptanedione-3,5; 2-acétylcyclohexanone (*Cy-acac*); 2,2,6,6-tétraméthyl-3,5-heptanedione (*t-Bu-acac*); 2,2,7-triméthyl-3,5-octanedione; 1,1,1,5,5,5-hexafluoro-2,4-pentanedione (*F-acac*)]; benzoylacétone; dibenzoyl-méthane; 3-méthyl-2,4-pentadione; 3-acétyl-pentane-2-one; 3-acétyl-2-hexanone; 3-acétyl-2-heptanone; 3-acétyl-5-méthyl-2-hexanone; stéaroylbenzoylméthane; octanoylbenzoylméthane; 4-t-butyl-4'-méthoxy-dibenzoylméthane; 4,4'-diméthoxy-dibenzoylméthane ; 4,4'-di-tert-butyl-dibenzoylméthane et 2,4-undécanedione,

Pour atteindre cet objectif, les inventeurs ont eu le mérite de mettre en évidence, de manière tout à fait surprenante et inattendue, que l'utilisation d'un complexe ou sel métallique de formule **(1)** qui est à base de zinc permet de catalyser la réaction entre un isocyanate et un alcool, étape clé pour la préparation de polymères polyuréthanes.

Il est à noter qu'au moins une partie du caractère inventif de l'invention, tient à la sélection judicieuse et avantageuse des associations délimitées de composés métalliques C selon l'invention utilisées à titre de catalyseur.

Selon un autre mode de réalisation préféré le catalyseur **C** est un complexe ou sel métallique choisi parmi le groupe constitué par les composés de formules **(8)** à **(9)** suivantes:
**(8) :** [Zn(TMOD)₂(N,N'-diméthyl-éthylènediamine)] avec *(TMOD)* = l'anion 2,2,7-triméthyl-3,5-octanedionato ou l'anion énolate de la 2,2,7-triméthyl-3,5-octanedione, et
**(9) :** [Zn(TMOD)₂ (N-propyl-éthylènediamine)] avec *(TMOD)* = l'anion 2,2,7-triméthyl-3,5-octanedionato ou l'anion énolate de la 2,2,7Htriméthyl-3,5-octanedione.

La quantité de catalyseur est avantageusement déterminée de manière à ce que le rapport molaire Zn/fonction isocyanate (Zn/NCO) soit compris entre 1/1000

Selon un autre mode de réalisation préféré, le procédé selon l'invention est caractérisé en ce que l'étape 1) consiste à faire réagir en l'absence d'humidité et en présence d'une quantité efficace du catalyseur **C** selon l'invention et tel que décrit ci-dessus, au moins un composé **B** qui est un isocyanate choisi parmi le groupe constitué par: les monoisocyanates, les diisocyanates, les polyisocyanates et leurs mélanges, et au moins un composé **D** qui est un alcool choisi parmi le groupe constitué par: les monoalcools, les diols, les polyols et leurs mélanges.

A titre d'illustration de composés **B** utile selon l'invention et ayant au moins une fonction isocyanate, on peut citer les mono, di ou polyisocyanates aromatiques, cycliques saturés ou aliphatiques bien connus de l'homme du métier, ainsi que les mélanges de ces composés.

Selon l'usage courant en chimie, lorsqu'une fonction a donné son nom a une famille de composés (en d'autres termes quand une fonction sert d'éponyme a une famille de produits, comme c'est le cas pour les isocyanates), on définit le caractère aromatique ou aliphatique selon le point d'attache de la fonction considérée.

Lorsqu'un isocyanate est situé sur un carbone de nature aliphatique, alors on considère que le compose isocyanate est lui-même de nature aliphatique. De même, lorsqu'une fonction isocyanate est rattachée au squelette par l'intermédiaire d'un carbone de nature aromatique, alors on désignera l'ensemble du monomère par l'expression isocyanate aromatique. Ainsi on considère comme:
- "aromatique", toute fonction isocyanate dont le point d'attache de l'azote est un maillon d'un cycle aromatique; et
- "aliphatique", toute fonction isocyanate dont le point d'attache de l'azote est un carbone d'hybridation sp³.

A titre d'exemples d'isocyanates aromatiques, on peut citer le diphénylméthane diisocyanate (MDI), notamment le diphénylméthane-4,4'-diisocyanate, le diphénylméthane-2,4'-diisocyanate, le toluène diisocyanate (TDI), notamment le toluène-2,4-diisocyanate et le toluène-2,6-diisocyanate.

A titre d'exemples d'isocyanates aliphatiques, on peut citer l'hexaméthylène diisocyanate (HMDI), le 1,3-tétraméthylxylylène diisocyanate, l'isophorone diisocyanate et le dicyclohexaméthylméthane diisocyanate.

A titre d'exemples de d'isocyanates cycloaliphatiques, on peut citer l'isophorone diisocyanate (IPDI).

Pour la préparation d'un polymère polyuréthane linéaire, on peut ainsi, de façon classique, faire réagir un diisocyanate et un diol. Les réactions en cause peuvent se développer selon de nombreuses variantes: il doit intervenir au moins deux réactifs de type différent (isocyanate/alcool), ces réactifs peuvent être mono ou difonctionnels.

A titre d'exemple de composé **D** ayant au moins une fonction hydroxyle, on peut citer, sans intention de s'y limiter, les polyols comme le glycérol, le polyglycérol, le glycol, le propylène glycol, les glycols comprenant de 2 à 10 atomes de carbone, de préférence de 2 à 6 atomes de carbone tels que l'éthylène glycol, le diéthylène glycol, le 1,4 butanediol, le 1,5 pentanediol, le 1,6 hexanediol, le 1,10-décanediol, le 1,3-propanediol, le dipropylène glycol polyéthylène glycol, le polypropylène glycol, le néopentylglycol, le pentaérythritol, l'hydroxypivalate de néopentylglycol, le dipentaérythritol, le triméthylol- propane, le butyl-2 éthyl-2 propanediol-1,3, le sorbitol, le mannitol, le xylitol et le mésoérythritol. On peut également utiliser des esters de ces diols ou des polyesters polyols ainsi que des polyol polyéthers.

De manière connue, les polyesters polyols sont généralement choisis parmi les polyesters polyols aliphatiques et aromatiques, et les mélanges de ces composés.

A titre d'exemple, on peut citer les polyesters polyols résultant de la condensation de polyols aliphatiques, cycliques ou aromatiques tels que le 1,2-éthanediol, le 1,2-propanediol, le 1,3-propanediol, le glycérol, le triméthylolpropane, le 1,6-hexanediol, le 1,2,6-hexanetriol, le butènediol, le sucrose, le glucose, le sorbitol, le pentaérythritol, le mannitol, la triéthanolamine, la N-méthyl diéthanolamine et les mélanges de ces composés avec un diacide carboxylique tel que l'acide 1,6-hexanedioïque, l'acide dodécanedioïque, l'acide azélaïque, l'acide sébacique, l'acide adipique, l'acide 1,18-octadécanedioïque, l'acide phtalique, l'acide succinique et les mélanges de ces diacides, un anhydride insaturé tel que l'anhydride maléique ou phtalique, ou un homopolymère de lactone telle que la ε-caprolactone.

Les polyesters polyols sont généralement obtenus par l'utilisation d'un excès d'alcool di- ou polyfonctionnel dans leur polyestérification avec des diacides ou des anhydrides carboxyliques.

Les polyols polyéthers sont en général obtenus par la polyaddition, anionique ou cationique, de monomères cycliques, comme l'oxyde d'éthylène, l'oxyde de propylène ou encore le tétrahydrofurane.

Les masses molaires des polyols polyéthers utilisés dans la synthèse de polyuréthanes varient en général de 250 à 8000. Leur fonctionnalité peut aller de 2 à 7 en fonction de la nature de la molécule utilisée comme amorceur. Les groupements terminaux de ces diols polyéthers peuvent être primaires ou secondaires.

Selon un autre mode de réalisation préféré, le procédé selon l'invention est caractérisé en ce que l'étape 1) est effectuée en présence d'un agent porogène.

De nombreux agents porogènes sont bien connus dans la technique et on les utilise à des quantités variées selon la taille cellulaire désirée dans le produit final qui est une mousse polyuréthane. Le plus économique de ces agents est l'eau mais on utilise souvent, seuls ou mélangés avec de l'eau, des alcanes à courtes chaînes halogénées portant du chlore et/ou du fluor. Les agents porogènes sont souvent utilisés à des quantités s'élevant jusqu'à 50 % du poids du polyol.

L'invention concerne aussi les nouveaux composés de formule **(8)** à **(9)** :
**(8) :** [Zn(TMOD)₂(N,N'-diméthyl-éthylènediamine)] avec *(TMOD)* = l'anion 2,2,7-triméthyl-3,5-octanedionato ou l'anion énolate de la 2,2,7-triméthyl-3,5-octanedione, et
**(9) :** [Zn(TMOD)₂ (N-propyl-éthylènediamine)] avec *(TMOD)* = l'anion 2,2,7-triméthyl-3,5-octanedionato ou l'anion énolate de la 2,2,7-triméthyl-3,5-octanedione.

Bien entendu diverses modifications peuvent être apportées par l'homme de l'art aux procédés qui viennent d'être décrits uniquement à titre d'exemples non limitatifs sans sortir du cadre de l'invention.

D'autres avantages et caractéristiques de la présente invention apparaîtront à la lecture des exemples suivants donnés à titre illustratif nullement limitatif.

### EXEMPLES

### 1-Catalyseurs

### - Catalyseur (6): [Zn(TMOD)₂] avec (TMOD) = l'anion 2,2,7-triméthyl-3,5-octanedionato ou l'anion énolate de la 2,2,7-triméthyl-3,5-octanedione

A une solution de 0.21 mol d'hydroxyde de potassium dans 88 g d'éthanol est ajoutée 0.21 mol de 2,2,7-triméthyl-3,5-octanedione à 95% en 1h à 20°C. A la solution homogène est ajoutée ensuite une solution de 0.105 mol de chlorure de zinc anhydre dans 16 g d'éthanol en 1h. Le mélange est agité pendant 3h à 30°C puis 23 g d'heptane sont ajoutés et la suspension est refroidie à 6°C puis filtrée. Le solide est rincé par 100 g d'heptane et le filtrat est évaporé jusqu'à 90°C sous 4 mbar pour donner 46 g de bis(2,2,7-triméthyl-3,5-octanedionate) de zinc sous forme d'un miel épais.
Zn calc. : 15.14 %pds, Zn trouvé : 14.50 %pds
IR (nm) : 2953, 1574, 1509, 1410, 1162.

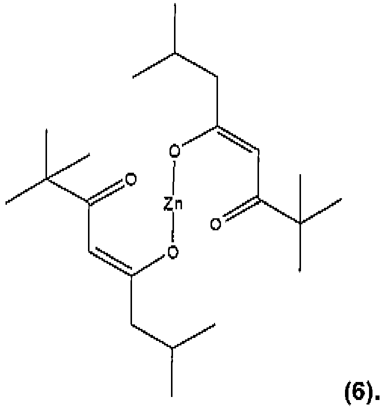

### - Catalyseur (7): [Zn(UDD)₂] avec (UDD) = l'anion 2,4-undécanedionato ou l'anion énolate de la 2,4-undécanedione

A une solution de 30 mmol de méthylate de sodium dans 10g d'éthanol à 70°C sont ajoutées 30 mmol de 2,4-undécanedione puis après 1h une solution de 15 mmol de chlorure de zinc anhydre dans 2.5 g d'éthanol. Le mélange est agité pendant 3h à 70°C puis 20 g d'heptane sont ajoutés et la suspension est refroidie à 6°C puis filtrée. Le solide est rincé par 20 g d'heptane et le filtrat est évaporé jusqu'à 90°C sous 4 mbar pour donner 6.2 g de bis(2,4-undécanedionate) de zinc sous forme d'un liquide jaune pâle peu visqueux,
Zn calc. : 15.14 %pds, Zn trouvé : 15.74 %pds
IR (nm) : 2992, 1575, 1511, 1389, 1015, 774.

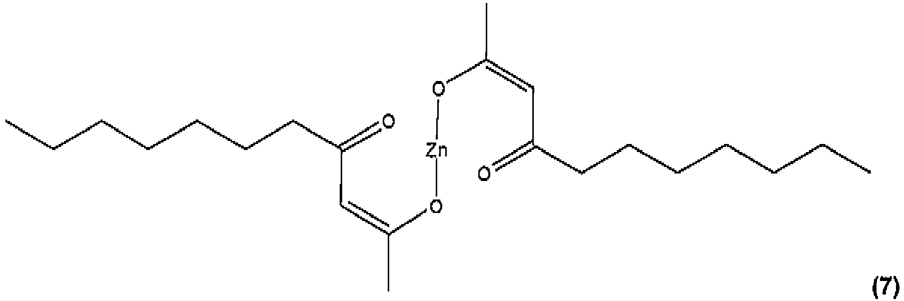

Ainsi que 2 complexes du Zn(TMOD)₂ avec un dérivé de l'éthylènediamine :
- [Zn(TMOD)₂(N,N'-diméthyl-éthylènediamine)] **(8)** préparé de la manière suivante;
   A une solution de 1 équivalent molaire de Zn(TMOD)₂ (exemple 10 mmol = 4.4g) dans l'éther diisopropylique (exemple 30 ml) est ajouté 1 équivalent molaire de N,N'-diméthyléthylènediamine (exemple 10 mmol = 0.9g) en 5 min. Après 30 min, la solution limpide est évaporée pour donner une huile visqueuse correspondant au complexe attendu (exemple 10 mmol = 5.2 g, 100%).
   RMN 1H (C₆D₁₄) : 5.23 (s, 2H), 2.77 (s, 4H), 2.41 (s, 6H), 2.04 (m, 2H), 1.94 (d, 4H), 1.08 (s, 18H), 0.94 (d, 12H).
- [Zn(TMOD)₂ (N-propyl-éthylènediamine)] **(9)** préparé de la manière suivante:
   A une solution de 1 équivalent molaire de Zn(TMOD)₂ (exemple 10 mmol = 4.4g) dans l'éther diisopropylique (exemple 30 ml) est ajouté 1 équivalent molaire de N-propyléthylènediamine (exemple 10 mmol = 1.02g) en 5 min. Après 30 min, la solution trouble est évaporée pour donner un solide blanc correspondant au complexe attendu (exemple 10 mmol = 5.4 g, 100%).
   RMN 1H (C₆D₁₄) : 5.23 (s, 2H), 2.86 (m, 2H), 2.74 (m, 2H), 2.62 (t, 2H), 2.03 (m, 2H), 1.93 (d, 4H), 1.56 (sext, 2H), 1.07 (s, 18H), 0.92 (d, 12H), 0.86 (t, 3H), et
   - Catalyseur **(3):** [Zn(*t-Bu-acac*)₂] avec *(t-Bu-acac)* = l'anion 2,2,6,6-tétraméthyl-3,5-heptanedionato ou l'anion énolate de la 2,2,6,6-tétraméthyl-3,5-heptanedione - CAS 14363-14-5 - fourni par la société Sigma-Aldrich - MM= 431.9 g/mol en solution à 30% dans l'acétate d'éthyle ou l'acétate de butyle après chauffage.

### 2-Conditions opératoires :

On prépare une formulation composée d'un prépolymère d'hexaméthylènediisocyanate avec 0.522 mol NCO/100g. Dans un réacteur, on introduit 10 g de ce polymère dans 25 g de xylène (sous forme d'une solution préparée préalablement), puis 6.8 g de 2-éthylhexanol (1 mol d'OH/mol NCO).

On chauffe et lorsque la température du milieu réactionnel atteint 40°C on introduit :
- soit 5g de xylène pour la réaction non catalysée
- soit 5 g d'une solution du catalyseur choisi dans le xylène.

Ce qui correspond au t=0 de la réaction (déclenchement du chronomètre).

Différents catalyseurs ont été testés :
- [Zn(TMOD)₂] avec (TMOD) = l'anion 2,2,7-triméthyl-3,5-octanedionato ou l'anion énolate de la 2,2,7-triméthyl-3,5-octanedione **(6)**
- [Zn(UDD)₂] avec (UDD) = l'anion 2,4-undécanedionato ou l'anion énolate de la 2,4-undécanedione **(7)**

Ainsi que 2 complexes du Zn(TMOD)₂ avec un dérivé de l'éthylènediamine :
- [Zn(TMOD)₂(N,N'-diméthyl-éthylènediamine)] **(8)**
- [Zn(TMOD)₂ (N-propyl-éthylènediamine)] **(9)**

A titre comparatif ont été également testés deux catalyseurs à base d'étain:
- le dilaurate de dibutylétain (DLDBE).
- le dilaurate de dioctylétain (DLDOE), et
- le [Zn(acac)₂] ou diacétylacétonate de zinc.

L'activité des nouveaux catalyseurs est comparée à celle du dilaurate de dibutyl étain. Dans le tableau suivant, un équivalent de dilauraurate de dibutyl étain correspond à 1.58 × 10⁻⁵ mol d'étain pour 10g de prépolymère d'hexaméthylènediisocyanate. Le potentiel catalytique est évalué par le taux de transformation des fonctions isocyanates à t=10 minutes. La disparition des fonctions isocyanates est suivie par analyse Infra Rouge.

Les quantités de catalyseur utilisées et les résultats obtenus sont détaillés dans le tableau suivant.

**Tableau 1**

| **Essai** | **Catalyseur** | **Equivalent molaire** | **TT des fonctions N=C-O (%) à t=10 min** |
|---|---|---|---|
| **Comparatif 1** | DLDBE | 1 | 99,2 |
| **Comparatif 2** | DLDOE | 1 | 98,9 |
| **Comparatif 3** | [Zn(aac)₂] | 3 | 62,2 |
| **Comparatif 4** | [Zn (TMOD)₂] **(6)** | 3 | **76,0** |
| **Comparatif 5** | [Zn(UDD)₂] **(7)** | 3 | **81,1** |
| **Invention 6** | [Zn(TMOD)₂ (N,N'-diméthyl-éthylènediamine)] **(8)** | 3 | **91,0** |
| **Invention 7** | [Zn(TMOD)₂ (N-propyl-éthylènediamine)] **(9)** | 3 | **88,8** |

Les catalyseurs selon l'invention permettent d'obtenir un bon taux de transformation des fonctions isocyanates après 10 minutes de réaction. Ils présentent une activité qui est supérieure de **46%** (pour le [Zn(TMOD)₂(N,N'-diméthyl-éthylènediamine)]) à celle du [Zn(acac)₂].

Les catalyseurs selon l'invention représentent donc une alternative efficace aux catalyseurs à base d'étain pour la réaction entre un isocyanate et un alcool.

## Revendications

1. Procédé pour préparer un composé **A** ayant au moins une fonction uréthane comprenant une étape 1) consistant à faire réagir un composé **B** ayant au moins une fonction isocyanate avec un composé **D** ayant au moins une fonction hydroxyle en présence d'une quantité catalytiquement efficace d'au moins un catalyseur **C caractérisé en ce que** ledit catalyseur **C** est un complexe ou sel métallique de formule **(1)** suivante:
[Zn(L1)ₗ₁(Y)x] **(1)**
dans laquelle:
- l₁ = 2, et x= 1 ou 2,
- le symbole Y représente une amine ou une diamine, et
- le symbole L¹ est un anion β-dicétonato dérivé d'une β-dicétone de formule R¹COCHR²COR³ **(2)** et ladite β-dicétone est choisie parmi le groupe constitué par les β-dicétones: l'hexanedione-2,4; heptanedione-2,4; heptanedione-3,5; l'éthyl-3 pentanedione-2,4; méthyl-5 hexanedione-2,4; octanedione-2,4; octanedione-3,5; diméthyl-5,5 hexanedione-2,4; méthyl-6 heptanedione-2,4; diméthyl-2, 2 nonanedione-3,5; diméthyl-2,6 heptanedione-3,5; 2-acétylcyclohexanone (*Cy-acac*); 2,2,6,6-tétraméthyl-3,5-heptanedione (*t-Bu-acac*); 2,2,7-triméthyl-3,5-octanedione ;1,1,1,5,5,5-hexafluoro-2,4-pentanedione (*F-acac*)]; benzoylacétone; dibenzoyl-méthane; 3-méthyl-2,4-pentadione; 3-acétyl-pentane-2-one; 3-acétyl-2-hexanone; 3-acétyl-2-heptanone; 3-acétyl-5-méthyl-2-hexanone; stéaroylbenzoylméthane; octanoylbenzoylméthane; 4-t-butyl-4'-méthoxy-dibenzoylméthane; 4,4'-diméthoxy-dibenzoylméthane ; 4,4'-di-tert-butyl-dibenzoylméthane et 2,4-undécanedione.

2. Procédé selon l'une quelconque des revendications précédentes **caractérisé en ce que** le composé **A** ayant au moins une fonction uréthane est un polyuréthane, le composé **B** ayant au moins une fonction isocyanate est un diisocyanate et le composé **D** ayant au moins une fonction hydroxyle est un polyol.

3. Procédé selon la revendication 1 dans lequel l'étape 1) consiste à faire réagir en l'absence d'humidité et en présence d'une quantité catalytiquement efficace du catalyseur **C** selon la revendication 1, au moins un composé **B** qui est un isocyanate choisi parmi le groupe constitué par: les monoisocyanates, les diisocyanates et leurs mélanges, et au moins un composé **D** qui est un alcool choisi parmi le groupe constitué par: les monoalcools, les diols, les polyols et leurs mélanges.

4. Procédé selon l'une des revendications précédentes **caractérisé en ce que** le composé **B** est un diisocyanate choisi parmi le groupe constitué par les composés suivants: le diphénylméthane diisocyanate (MDI), notamment le diphénylméthane-4,4'-diisocyanate, le diphénylméthane-2,4'-diisocyanate, le toluène diisocyanate (TDI), notamment le toluène-2,4-diisocyanate, le toluène-2,6-diisocyanate, l'hexaméthylène diisocyanate (HMDI), le 1,3-tétraméthylxylylène diisocyanate, l'isophorone diisocyanate, dicyclohexaméthylméthane diisocyanate et l'isophorone diisocyanate (IPDI).

5. Procédé selon la revendication 1 **caractérisé en ce que** le composé **D** est un polyester polyol.

6. Procédé selon la revendication 1 ou 3 **caractérisé en ce que** ledit catalyseur **C** est un complexe ou sel métallique choisi parmi le groupe constitué par les composés de formules **(8)** et **(9)** suivantes:
**(8) :** [Zn(TMOD)₂(N,N'-diméthyl-éthylènediamine)] avec *(TMOD)* = l'anion 2,2,7-triméthyl-3,5-octanedionato ou l'anion énolate de la 2,2,7-triméthyl-3,5-octanedione, et
**(9) :** [Zn(TMOD)₂ (N-propyl-éthylènediamine)] avec *(TMOD)* = l'anion 2,2,7-triméthyl-3,5-octanedionato ou l'anion énolate de la 2,2,7-triméthyl-3,5-octanedione

7. Procédé selon l'une des revendications précédentes **caractérisé en ce que** l'étape 1) est effectuée en présence d'un agent porogène.

8. Composés de formule **(8)** et **(9)** :
**(8) :** [Zn(TMOD)₂(N,N'-diméthyl-éthylènediamine)] avec *(TMOD)* = l'anion 2,2,7-triméthyl-3,5-octanedionato ou l'anion énolate de la 2,2,7-triméthyl-3,5-octanedione, et
**(9) :** [Zn(TMOD)₂ (N-propyl-éthylènediamine)] avec *(TMOD)* = l'anion 2,2,7-triméthyl-3,5-octanedionato ou l'anion énolate de la 2,2,7-triméthyl-3,5-octanedione.

## Patentansprüche

1. Verfahren zur Herstellung einer Verbindung **A** mit mindestens einer Urethanfunktion, umfassend einen Schritt 1), der in der Umsetzung einer Verbindung **B** mit mindestens einer Isocyanatfunktion mit einer Verbindung **D** mit mindestens einer Hydroxylfunktion in Gegenwart einer katalytisch wirksamen Menge mindestens eines Katalysators **C** besteht, **dadurch gekennzeichnet, dass** es sich bei dem Katalysator **C** um einen Metallkomplex oder ein Metallsalz der folgenden Formel **(1)** handelt:
[Zn(L¹)ₗ₁(Y)ₓ] (**1**)
in der:
- l₁ = 2 und x = 1 oder 2,
- das Symbol Y für ein Amin oder ein Diamin steht
und
das Symbol L¹ für ein β-Diketonato-Anion steht, das sich von einem β-Diketon der Formel R¹COCHR²COR³ (**2**) ableitet und das β-Diketon aus der Gruppe bestehend aus den β-Diketonen: 2,4-Hexandion; 2,4-Heptandion; 3,5-Heptandion; 3-Ethyl-2,4-pentan-dion; 5-Methyl-2,4-hexandion; 2,4-0ctandion; 3,5-Octandion; 5,5-Dimethyl-2,4-hexandion; 6-Methyl-2,4-heptandion; 2,2-Dimethyl-3,5-nonandion; 2,6-Dimethyl-3,5-heptandion; 2-Acetylcyclohexanon (*Cy-acac*)*;* 2,2,6,6-Tetramethyl-3,5-heptandion (*t*-*Bu-acac*); 2,2,7-Trimethyl-3,5-octandion; 1,1,1,5,5,5-Hexafluor-2,4-pentandion (*F-acac*); Benzoylaceton; Dibenzoylmethan; 3-Methyl-2,4-pentandion; 3-Acetylpentan-2-on; 3-Acetyl-2-hexanon; 3-Acetyl-2-heptanon; 3-Acetyl-5-methyl-2-hexanon; Stearoylbenzoylmethan; Octanoylbenzoylmethan; 4-(t-Butyl)-4'-methoxydibenzoylmethan; 4,4'-Dimethoxydibenzoylmethan; 4,4'-Di(tert-butyl)dibenzoylmethan und 2,4-Undecandion ausgewählt ist.

2. Verfahren nach einem der vorhergehenden Ansprüche, **dadurch gekennzeichnet, dass** es sich bei der Verbindung **A** mit mindestens einer Urethanfunktion um ein Polyurethan handelt, es sich bei der Verbindung **B** mit mindestens einer Isocyanatfunktion um ein Diisocyanat handelt und es sich bei der Verbindung **D** mit mindestens einer Hydroxylfunktion um ein Polyol handelt.

3. Verfahren nach Anspruch 1, wobei der Schritt 1) darin besteht, dass man in Abwesenheit von Feuchtigkeit und in Anwesenheit einer katalytisch wirksamen Menge des Katalysators **C** nach Anspruch 1, mindestens eine Verbindung **B,** bei der es sich um ein Isocyanat aus der Gruppe bestehend aus Monoisocyanaten, Diisocyanaten und Mischungen davon handelt, und mindestens eine Verbindung **D,** bei der es sich um einen Alkohol, der aus der Gruppe bestehend aus Monoalkoholen, Diolen, Polyolen und Mischungen davon ausgewählt ist, handelt, umsetzt.

4. Verfahren nach einem der vorhergehenden Ansprüche, **dadurch gekennzeichnet, dass** es sich bei der Verbindung **B** um ein Diisocyanat aus der Gruppe bestehend aus den folgenden Verbindungen handelt: Diphenylmethandiisocyanat (MDI), insbesondere 4,4'-Diphenylmethandiisocyanat oder 2,4'-Diphenylmethandiisocyanat, Toluoldiisocyanat (TDI), insbesondere 2,4-Toluoldiisocyanat oder 2,6-Toluoldiisocyanat, Hexamethylendiisocyanat (HMDI), 1,3-Tetramethylxylylendiisocyanat, Isophorondiisocyanat, Dicyclohexamethylmethandiisocyanat und Isophorondiisocyanat (IPDI).

5. Verfahren nach Anspruch 1, **dadurch gekennzeichnet, dass** es sich bei der Verbindung **D** um ein Polyesterpolyol handelt.

6. Verfahren nach einem der Ansprüche 1 bis 3, **dadurch gekennzeichnet, dass** es sich bei dem Katalysator **C** um einen Metallkomplex oder ein Metallsalz aus der Gruppe bestehend aus den Verbindungen der folgenden Formeln **(8)** und **(9)** handelt:
(**8**): Zn(*TMOD*)₂(N,N'-Dimathylethylendiamin)], mit *(TMOD)* = das 2,2,7-Trimethyl-3,5-octandionato-Anion oder das Enolat-Anion von 2,2,7-Trimethyl-3,5-octandion, und
(**9**): [Zn(*TMOD*)₂(N-Propylethylendiamin)], mit *(TMOD)* = das 2,2,7-Trimethyl-3,5-octandionato-Anion oder das Enolat-Anion von 2,2,7-Trimethyl-3,5-octandion.

7. Verfahren nach einem der vorhergehenden Ansprüche, **dadurch gekennzeichnet, dass** Schritt 1) in Gegenwart eines Porenbildners durchgeführt wird.

8. Verbindungen der Formel **(8)** und **(9):**
(**8**): [Zn(*TMOD*)₂(N,N'-Dimethylethylendiamin)], mit *(TMOD)* = das 2,2,7-Trimethyl-3,5-octandionato-Anion oder das Enolat-Anion von 2,2,7-Trimethyl-3,5-octandion, und
(**9**): [Zn(*MOD*)₂(N-Propylethylendiamin)], mit *(TMOD)* = das 2,2,7-Trimethyl-3,5-octandionato-Anion oder das Enolat-Anion von 2,2,7-Trimethyl-3,5-octan-dion.

## Claims

1. Process for preparing a compound **A** having at least one urethane functional group, comprising a stage 1) which consists in reacting a compound **B,** having at least one isocyanate functional group, with a compound **D,** having at least one hydroxyl functional group, in the presence of a catalytically effective amount of at least one catalyst **C, characterized in that** said catalyst **C** is a metal complex or salt of following formula (**1**) :
[Zn(Lⁱ)ₗ₁(Y)ₓ] (**1**)
in which:
- l₁= 2, and x = 1 or 2,
- the symbol Y represents an amine or a diamine,
and
- the symbol L¹ is a β-diketonato anion derived from a β-diketone of formula R¹COCHR²COR³ (**2**) and said β-diketone is chosen from the group consisting of the β-diketones: 2,4-hexanedione; 2,4-heptanedione; 3,5-heptanedione; 3-ethyl-2,4-pentanedione; 5-methyl-2,4-hexanedione; 2,4-octanedione; 3,5-octanedione; 5,5-dimethyl-2,4-hexanedione; 6-methyl-2,4-heptanedione; 2,2-dimethyl-3,5-nonanedione; 2,6-dimethyl-3,5-heptanedione; 2-acetylcyclohexanone (*Cy-acac*)*;* 2,2,6,6-tetramethyl-3,5-heptanedione (*t-Bu-acac*); 2,2,7-trimethyl-3,5-octanedione; 1,1,1,5,5,5-hexafluoro-2,4-pentanedione (*F'-acac*); benzoylacetone; dibenzoylmethane; 3-methyl-2,4-pentanedione; 3-acetylpentane-2-one; 3-acetyl-2-hexanone; 3-acetyl-2-heptanone; 3-acetyl-5-methyl-2-hexanone; stearoylbenzoylmethane; octanoyl-benzoylmethane; 4-(t-butyl)-4'-methoxydibenzoylmethane; 4,4'-dimethoxydibenzoylmethane; 4,4'-di(tert-butyl)dibenzoylmethane and 2,4-undecanedione.

2. Process according to any one of the preceding claims, **characterized in that** the compound **A** having at least one urethane functional group is a polyurethane, the compound **B** having at least one isocyanate functional group is a diisocyanate and the compound **D** having at least one hydroxyl functional group is a polyol.

3. Process according to Claim 1, in which stage 1) consists in reacting, in the absence of moisture and in the presence of a catalytically effective amount of the catalyst **C** according to claim 1, at least one compound **B,** which is an isocyanate chosen from the group consisting of monoisocyanates, diisocyanates, polyisocyanates and their mixtures, and at least one compound **D,** which is an alcohol chosen from the group consisting of monoalcohols, diols, polyols and their mixtures.

4. Process according to one of the preceding claims, **characterized in that** the compound **B** is a diisocyanate chosen from the group consisting of the following compounds: diphenylmethane diisocyanate (MDI), in particular 4,4'-diphenylmethane diisocyanate or 2,4'-diphenylmethane diisocyanate, toluene diisocyanate (TDI), in particular 2,4-toluene diisocyanate or 2,6-toluene diisocyanate, hexamethylene diisocyanate (HMDI), 1,3-tetramethylxylylene diisocyanate, isophorone diisocyanate, dicyclohexamethylmethane diisocyanate and isophorone diisocyanate (IPDI).

5. Process according to Claim 1, **characterized in that** the compound **D** is a polyester polyol.

6. Process according to Claim 1 or 3, **characterized in that** said catalyst **C** is a metal complex or salt chosen from the group consisting of the compounds of following formulae (**8**) and (**9**):
(**8**): [Zn(*TMOD*)₂(N,N'-dimethylethylenediamine)], with *(TMOD)* = the 2,2,7-trimethyl-3,5-octanedionato anion or the enolate anion of 2,2,7-trimethyl-3,5-octanedione, and
(**9**): Zn(*TMOD*)₂(N-propylethylenediamine)], with *(TMOD)* = the 2,2,7-trimethyl-3,5-octanedionato anion or the enolate anion of 2,2,7-trimethyl-3,5-octanedione.

7. Process according to one of the preceding claims, **characterized in that** stage 1) is carried out in the presence of an expanding agent.

8. Compounds of formulae (**8**) and (**9**):
(**8**): [Zn(*TMOD*)₂(N,N'-dimethylethylenediamine)], with *(TMOD)* = the 2,2,7-trimethyl-3,5-octanedionato anion or the enolate anion of 2,2,7-trimethyl-3,5-octanedione, and
(**9**): [Zn(*TMOD*)₂(N-propylethylenediamine)], with *(TMOD)* = the 2,2,7-trimethyl-3,5-octanedionato anion or the enolate anion of 2,2,7-trimethyl-3,5-octanedione.
